# EUROPÄISCHE PATENTSCHRIFT

(11) **EP 1 197 486 B1**
(45) Veröffentlichungstag und Bekanntmachung des Hinweises auf die Patenterteilung: **15.10.2003**
(21) Anmeldenummer: 01120530.9
(22) Anmeldetag: 29.08.2001
(51) Int. Cl.: C07D 231/12

(54) **Verfahren zur Herstellung von 1H-Pyrazol-1-Carboxamidinen**
Process for the preparation of 1H-pyrazole-1-carboxamidines
Procédé pour la préparation des 1H-pyrazole-1-carboxamidines

(30) Priorität: 13.10.2000 DE 10050900
(43) Veröffentlichungstag der Anmeldung: 17.04.2002
(73) Patentinhaber: Degussa AG, 40474 Düsseldorf (DE)
(72) Erfinder: Fries, Guido, Dr., 45657 Recklinghausen (DE)

(56) Entgegenhaltungen:
- WO-A-97/30972
- US-A- 5 453 514
- BERNATOWICZ, MICHAEL S. ET AL: "1H-Pyrazole-1-carboxamidine hydrochloride an attractive reagent for guanylation of amines and its application to peptide synthesis" J. ORG. CHEM. (1992), 57(8), 2497-502 , XP001037257
- LEE, Y. ET AL: "1H-Pyrazole-1-carboxamidines: new inhibitors of nitric oxide synthase" BIOORG. MED. CHEM. LETT. (2000), 10(24), 2771-2774 , XP004225347

## Beschreibung

Die Erfindung betrifft ein Verfahren zur Herstellung von 1-Guanylpyrazolsäureadditionsprodukten der allgemeinen Formel 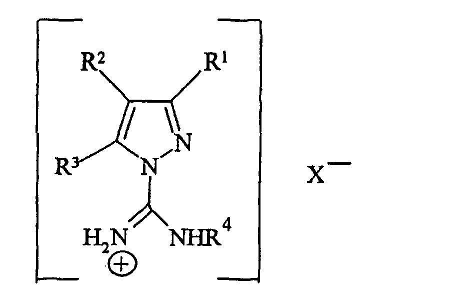 ,wobei
R¹, R² und R³ unabhängig voneinander ein Wasserstoffatom, eine verzweigte oder unver-zweigte Alkylgruppe mit 1 bis 6 Kohlenstoffatomen, eine Cycloalkylgruppe mit 3 bis 8 Kohlenstoffatomen oder eine unsubstituierte oder mit 1 bis 6 Alkylgruppen substituierte Arylgruppe mit insgesamt 6 bis 12 Kohlenstoffatomen,
R⁴ ein Wasserstoffatom oder eine unverzweigte oder verzweigte Alkylgruppe mit 1 bis 4 Kohlenstoffatomen und
X Cl oder Br
bedeutet,
durch Umsetzung von Pyrazol oder dessen Derivaten mit Cyanamid oder dessen Derivaten und gasförmigem Chlorwasserstoff oder Bromwasserstoff in aprotischen polaren Lösemittel, ausgewählt aus der Gruppe, die durch Sulfoxide, Sulfone, Sulfolane, Ketone, Polyether, Nitrile, 1,2- Dichlorethan, Dichlormethan, Methyl- tert.-butylether, Diphenylether, Diisopropylether, Anisol und Dimethoxyethan gebildet wird, und Aufarbeitung durch eine mechanische Trennoperation und gegebenenfalls eine thermische Nachbehandlung.

1-Guanylpyrazolsäureadditionsprodukte der oben genannten Formel mit X = Cl oder Br (1H-Pyrazol-1-carboxamidin-Hydrohalogenide und dessen Derivate) können als Nitrifikationsinhibitoren und für die Guanylierung von Aminen, wie zum Beispiel bei der Umsetzung von Ornithin zu Arginin, verwendet werden.

Synthesen von 1H-Pyrazol-1-carboxamidin-Hydrochlorid und dessen Derivaten aus Pyrazol beziehungsweise den verschieden substituierten Vertretern, Cyanamid und Säuren sind aus der Literatur bekannt. So wird in der deutschen Patentanmeldung DE 42 37 687 A1 ein Verfahren beschrieben, bei dem substituierte 1H-Pyrazole mit wässriger Cyanamidlauge unter Zusatz von mindestens äquimolaren Mengen einer Säure umgesetzt werden. Ein großer Nachteil dieses Verfahrens ist das Entstehen organisch belasteten Abwassers, das entsorgt werden muss.

In J. Org. Chem. 1992, 57, Seite 2497, wird die Herstellung von 1H-Pyrazol-1-carboxamidin-Hydrochlorid durch 2-stündiges Erhitzen von Pyrazol mit Cyanamid in einer 4 N p-Dioxan/HCl-Lösung unter Rückfluss beschrieben. Aufgrund der leichten Bildung von explosiven Peroxiden stellt die Verwendung von p-Dioxan als Lösemittel jedoch einen gravierenden Nachteil dar. Ebenso beschreibt die WO97/30972 diese Reaktion in THF/Dioxan als Lösengsmittel.

Aufgabe der Erfindung war es daher, ein Verfahren zur Herstellung von 1H-Pyrazol-1-carboxamidin-Hydrochlorid oder -Hydrobromid oder dessen Derivaten zu finden, das technisch einfach ist, bei dem auf p-Dioxan und andere leicht Peroxid-bildende Ether als Lösemittel verzichtet werden kann und wässrige Säure keine Verwendung findet, so dass keine aufwendig und kostenintensiv zu entsorgenden Abfälle anfallen.

Die Aufgabe wird erfindungsgemäß dadurch gelöst, dass die Umsetzung von Pyrazol oder dessen Derivaten mit Cyanamid oder dessen Derivaten in einem aprotischen, keine Peroxide bildenden Lösemittel durchgeführt und gasförmiger Chlorwasserstoff oder Bromwasserstoff verwendet wird.

Gegenstand der Erfindung ist daher ein Verfahren zur Herstellung von 1-Guanylpyrazolsäureadditionsprodukten der allgemeinen Formel 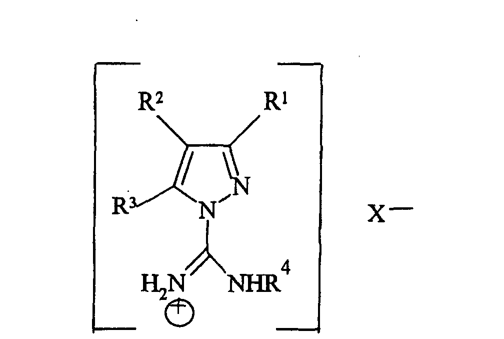 ,wobei
R¹, R² und R³ unabhängig voneinander ein Wasserstoffatom, eine verzweigte oder unverzweigte Alkylgruppe mit 1 bis 6 Kohlenstoffatomen, eine Cycloalkylgruppe mit 3 bis 8 Kohlenstoffatomen oder eine unsubstituierte oder mit 1 bis 6 Alkylgruppen substituierte Arylgruppe mit insgesamt 6 bis 12 Kohlenstoffatomen,
R⁴ ein Wasserstoffatom oder eine unverzweigte oder verzweigte Alkylgruppe mit 1 bis 4 Kohlenstoffatomen wie beispielsweise eine Methyl-, Ethyl-, Isopropyl- oder tert.-Butylgruppe
und X Cl oder Br
bedeutet,
durch Umsetzung von Pyrazol oder dessen Derivaten mit Cyanamid oder dessen Derivaten und gasförmigem Chlorwasserstoff oder Bromwasserstoff in Polaren aprotischen Lösemitteln, die aus der in Anspruch 1 aufgelisteten Gruppe ausgewählt werden.

Die Aufarbeitung erfolgt in der Regel durch eine mechanische Trennoperation, wie zum Beispiel Filtration, Membranfiltration, Dekantieren oder Zentrifugieren, und gegebenenfalls eine thermische Nachbehandlung, wie zum Beispiel eine Trocknung, vorzugsweise im Vakuum.

Das Verfahren zur Herstellung von lH-Pyrazol-1-carboxamidin-Hydrochlorid oder -Hydrobromid und dessen Derivaten umfasst damit insbesondere die Reaktions- und Verfahrensschritte:
- Umsetzung von Pyrazol beziehungsweise eines Pyrazolderivates mit Cyanamid beziehungsweise eines Cyanamidderivates und gasförmigem Chlorwasserstoff oder Bromwasserstoff,
- mechanische Abtrennung des anfallenden Carboxamidin-Hydrochlorids oder -Hydrobromids und
- gegebenenfalls thermische Behandlung des isolierten rohen Carboxamidin-Hydrochlorids oder Hydrobromids (insbesondere zur Entfernung anhaftender restlicher Lösemittel).

Für das erfindungsgemäße Verfahren eignen sich aprotische, polare Lösemittel. Beispiele geeigneter Lösemittel sind chlorierte Kohlenwasserstoffe wie zum Beispiel 1,2-Dichlorethan, Dichlormethan und Trichlormethan, Sulfoxide, Sulfone und Sulfolane wie zum Beispiel Dimethylsulfoxid, Diisopropylsulfon, Sulfolan, 2-Methylsulfolan, 3-Methylsulfolan und 2-Methyl-4-butylsulfolan, Ketone wie zum Beispiel Aceton, Methylethylketon, Methylisobutylketon oder Cyclohexanon, Ether wie zum Beispiel Methyl-tert.-butylether, Diphenylether, Diisopropylether, Anisol und Dimethoxyethan, Polyether wie zum Beispiel Diethylenglykoldimethylether sowie Nitrile wie zum Beispiel Acetonitril. Bevorzugt wird Dimethoxyethan verwendet.

Die Reaktionstemperaturen liegen zwischen -20 °C und 150 °C, bevorzugt zwischen 0 °C und 100 °C und besonders bevorzugt zwischen 60 °C und 90 °C. Eine Reaktionstemperatur von etwa 70 °C bis etwa 85 °C wird ganz besonders bevorzugt.

Die Umsetzung erfolgt bei einem Druck von 0,1 bar bis 50 bar, vorzugsweise bei einem Druck von 1 bar bis 10 bar. Besonders bevorzugt erfolgt die Umsetzung bei circa 1 bar (Umgebungsdruck). Die eingeleitete Gasmenge an Chlorwasserstoff oder Bromwasserstoff wird so angepasst, dass möglichst quantitativer Verbrauch erfolgt und das Abgas möglichst arm an unumgesetzten Chlorwasserstoff oder Bromwasserstoff ist. Vorzugsweise wird Chlorwasserstoff verwendet.

Das Verfahren kann diskontinuierlich oder kontinuierlich durchgeführt werden.

Die Reaktion ist bei diskontinuierlicher Fahrweise in etwa 15 Minuten bis 8 Stunden, vorzugsweise in 30 Minuten bis 4 Stunden, im Besonderen in 45 Minuten bis 60 Minuten, beendet. Es wurde damit überraschend gefunden, das die Verwendung von gasförmigem Chlorwasserstoff oder Bromwasserstoff, insbesondere Chlorwasserstoff, der durch die Reaktionslösung geleitet wird, vorzugsweise zu einer Halbierung der in J. Org. Chem. 1992, 57 Seite 2497 beschriebenen Reaktionszeit und damit zu einer deutlichen Erhöhung der Raum/Zeit-Ausbeute führt.

Anschließend wird das Reaktionsgemisch abgekühlt, der ausgefallene Feststoff abgetrennt, wozu vorzugsweise eine mechanische Trennoperation wie eine normale Filtration oder Membranfiltration oder eine Abtrennung durch Zentrifugieren oder Dekantieren angewendet werden kann, und gegebenenfalls einer thermischen Nachbehandlung, vorzugsweise einer Trocknung, insbesondere im Vakuum, unterworfen. Eine weitere Reinigung ist in der Regel nicht erforderlich. Es kann aber auch in besonderen Fällen, zum Beispiel bei besonders hohen Reinheitsanforderungen, eine Umkristallisation oder eine andere geeignete an sich bekannte Reinigungsoperation durchgeführt werden.

Die folgenden Beispiele sollen das erfindungsgemäße Verfahren näher erläutern, nicht jedoch auf die speziell genannten Umstände einschränken.

### Beispiel 1: 1H-Pyrazol-1-carboxamidin-Hydrochlorid

In einem 500 ml-Vierhalskolben mit Gaseinleitungs- und Gasableitungsrohr, Innenthermometer und Rückflusskühler, dem jeweils zwei Sicherheitswaschflaschen vor- und nachgeschaltet sind, werden 34,1 g (0,5 Mol) Pyrazol und 21,0 g (0,5 Mol) Cyanamid in 330 ml Dimethoxyethan gelöst. Bei 80°C wird für circa 50 Minuten trockenes Chlorwasserstoff-Gas mit einer Geschwindigkeit von circa 15 l/h in die Reaktionslösung eingeleitet. Anschließend lässt man die Lösung abkühlen und filtriert den ausgefallenen Feststoff ab. Nach dem Trocknen im Vakuum (100 mbar, 80 °C) erhält man lH-Pyrazol-1-carboxamidin-Hydrochlorid in Form eines farblosen, feinkristallinen Feststoffes. Ausbeute: 70,6 g (96 %)
Schmelzpunkt: 160 - 168 °C (Literatur: 165 - 166 °C)
¹³C-NMR (DMSO-d₆) δ 152.3, 146.0, 131.5, 111.9

### Beispiel 2: 3,5-Dimethyl-lH-pyrazol-1-carboxamidin-Hydrochlorid

Die Darstellung und Aufarbeitung erfolgt analog zu Beispiel 1. 48,1 g (0,5 Mol) 3,5-Dimethylpyrazol und 21,0 g (0,5 mol) Cyanamid werden in 330 ml Dimethoxyethan gelöst. Bei 80 °C wird 50 Minuten gasförmiger Chlorwasserstoff eingeleitet. Man erhält 3,5-Dimethyl-1H-pyrazol-1-carboxamidin-Hydrochlorid als farblosen, kristallinen Feststoff.
Ausbeute: 82 g (94 %)
Der Schmelzpunkt beträgt 148 - 150 °C (Literatur: 150 °C)

## Patentansprüche

1. Verfahren zur Herstellung von 1-Guanylpyrazolsäureadditionsprodukten der allgemeinen Formel 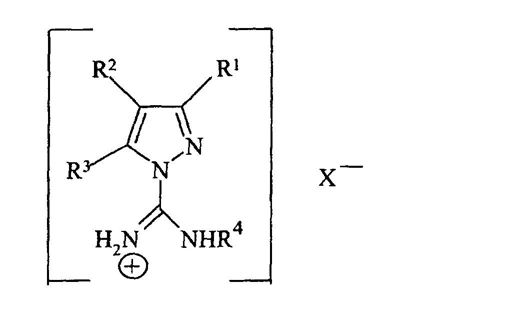 ,wobei
R¹, R² und R³ unabhängig voneinander ein Wasserstoffatom, eine verzweigte oder unverzweigte Alkylgruppe mit 1 bis 6 Kohlenstoffatomen, eine Cycloalkylgruppe mit 3 bis 8 Kohlenstoffatomen oder eine unsubstituierte oder mit 1 bis 6 Alkylgruppen substituierte Arylgruppe mit insgesamt 6 bis 12 Kohlenstoffatomen,
R⁴ ein Wasserstoffatom oder eine unverzweigte oder verzweigte Alkylgruppe mit 1 bis 4 Kohlenstoffatomen
und X Cl oder Br
bedeutet,
**dadurch gekennzeichnet,**
**dass** die Umsetzung von Pyrazol oder dessen Derivaten mit Cyanamid oder dessen Derivaten und gasförmigem Chlorwasserstoff oder Bromwasserstoff in einem aprotischen, polaren Lösemittel, ausgewählt aus der Gruppe, die durch Sulfoxide, Sulfone, Sulfolane, Ketone, Polyether, Nitrile, 1,2-Dichlorethan, Dichlormethan, Methyltert.-butylether, Diphenylether, Diisopropylether, Anisol und Dimethoxyethan gebildet wird, durchgeführt wird.

2. Verfahren nach Anspruch 1,
**dadurch gekennzeichnet,**
**dass** die Umsetzung in Dimethylsulfoxid, Diisopropylsulfon, Sulfolan, 2-Methylsulfolan, 3-Methylsulfolan oder 2-Methyl-4-butylsulfolan durchgeführt wird.

3. Verfahren nach Anspruch 1,
**dadurch gekennzeichnet,**
**dass** die Umsetzung in Aceton, Methylethylketon, Methylisobutylketon oder Cyclohexanon durchgeführt wird.

4. Verfahren nach Anspruch 1,
**dadurch gekennzeichnet,**
**dass** die Umsetzung in Diethylenglykoldimethylether durchgeführt wird.

5. Verfahren nach Anspruch 1,
**dadurch gekennzeichnet,**
**dass** die Umsetzung in Acetonitril durchgeführt wird.

6. Verfahren nach einem der vorhergehenden Ansprüche,
**dadurch gekennzeichnet,**
**dass** man die Reaktionskomponenten bei einem Druck von 0,1 bis 50 bar, einer Reaktionstemperatur zwischen -20 °C und 150 °C und Reaktionszeiten zwischen 15 Minuten und 8 Stunden zur Umsetzung bringt.

7. Verfahren nach einem der vorhergehenden Ansprüche,
**dadurch gekennzeichnet,**
**dass** man das anfallende Carboxamidin-Hydrochlorid oder -Hydrobromid durch eine mechanische Trennoperation abtrennt und gegebenenfalls einer thermischen Nachbehandlung unterwirft.

8. Verfahren nach einem der vorhergehenden Ansprüche,
**dadurch gekennzeichnet,**
**dass** die Reaktion diskontinuierlich oder kontinuierlich durchgeführt wird.

## Claims

1. A process for preparing a 1-guanylpyrazole acid adduct of the formula 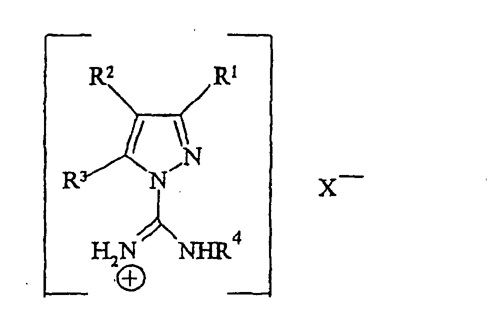 where
R¹, R² and R³ independently of one another are a hydrogen atom, a branched or straight-chain alkyl group having 1 to 6 carbon atoms, a cycloalkyl group having 3 to 8 carbon atoms or an aryl group unsubstituted or substituted by 1 to 6 alkyl groups and having a total of 6 to 12 carbon atoms,
R⁴ is a hydrogen atom or a straight-chain or branched alkyl group having 1 to 4 carbon atoms, and
X is Cl or Br,
**characterized in that** it comprises
carrying out the reaction of pyrazole or its derivative with cyanamide or its derivative and gaseous hydrogen chloride or hydrogen bromide in an aprotic, polar solvent selected from the group
consisting of sulphoxides, sulphones, sulpholanes, ketones, polyethers, nitriles, 1,2-dichloroethane, dichloromethane, methyl tert-butyl ether, diphenyl ether, diisopropyl ether, anisole and dimethoxyethane.

2. A process according to claim 1,
**characterized in that**
the reaction is carried out in dimethyl sulphoxide, diisopropyl sulphone, sulpholane, 2-methylsulpholane, 3-methylsulpholane or 2-methyl-4-butylsulpholane.

3. A process according to claim 1,
**characterized in that**
the reaction is carried out in acetone, methyl ethyl ketone, methyl isobutyl ketone or cyclohexanone.

4. A process according to claim 1,
**characterized in that**
the reaction is carried out in diethylene glycol dimethyl ether.

5. A process according to claim 1,
**characterized in that**
the reaction is carried out in acetonitrile.

6. A process according to any one of the preceding claims,
**characterized in that**
the reaction components are reacted at a pressure of from 0.1 to 50 bar, a reaction temperature between -20°C and 150°C and reaction times between 15 minutes and 8 hours.

7. A process according to any one of the preceding claims,
**characterized in that**
the carboxamidine hydrochloride or carboxamidine hydrobromide obtained is separated off in a mechanical separating operation and, if appropriate, subjected to thermal aftertreatment.

8. A process according to any one of the preceding claims,
**characterized in that**
the reaction is carried out batchwise or continuously.

## Revendications

1. Procédé de préparation de produits d'addition d'acide 1-guanylpyrazolique de formule générale : 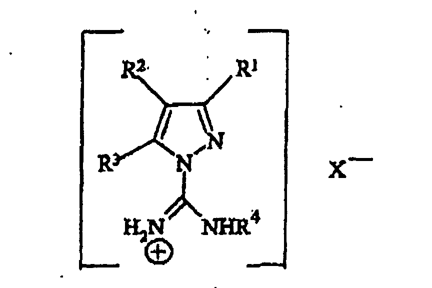 dans laquelle
R¹, R² et R³ indépendamment les uns des autres, signifient un atome d'hydrogène, un groupe alkyle ramifié ou non ramifié ayant de 1 à 6 atomes de carbone, un groupe cycloalkyle, ayant de 3 à 8 atomes de carbone, ou un groupe aryle non substitué ou substitué par 1 à 6 groupes allyle ayant au total de 6 à 12 atomes de carbone,
R⁴ signifie un atome de carbone ou un groupe alkyle non ramifié ou ramifié, ayant de 1 à 4 atomes de carbone, et
X signifie Cl ou Br,
**caractérisé en ce qu'**
on effectue la réaction du pyrazole ou de ses dérivés avec la cyanamide ou ses dérivés et de l'acide chlorhydrique gazeux ou de l'acide bromhydrique dans un solvant polaire aprotique choisi dans le groupe formé par les sulfoxydes, les sulfones, les sulfolanes, les cétones, les polyéthers, les nitriles, le 1,2-dichloroéthane, le dichlorométhane, l'éther tert.-butylique de méthyle, l'éther diphénylique, l'éther diisopropylique, l'anisol et le diméthoxyéthane.

2. Procédé selon la revendication 1,
**caractérisé en ce qu'**
on effectue la réaction dans le diméthylsulfoxyde, la diisopropylsulfane, le sulfolane, le 2-méthylsulfolane, le 3-méthylsulfolane, ou le 2-méthyl-4-butylsulfolane.

3. Procédé selon la revendication 1,
**caractérisé en ce qu'**
on effectue la réaction dans l'acétone, la méthyléthylcétone, la méthylisobutylcêtone, ou la cyclohexanone.

4. Procédé selon la revendication 1,
**caractérisé en ce qu'**
on effectue la réaction dans l'éther diméthylique de diéthylèneglycol.

5. Procédé selon la revendication 1,
**caractérisé en ce qu'**
on effectue la réaction dans l'acétonitrile.

6. Procédé selon l'une quelconque des revendications précédentes,
**caractérisé en ce qu'**
on met en réaction les composants de la réaction â une pression de 0,1 à 50 bars, â une température de réaction comprise entre -20°C et 150°C et pendant des durées de réaction comprises entre 15 minutes et 8 heures.

7. Procédé selon l'une quelconque des revendications précédentes,
**caractérisé en ce qu'**
on sépare le chlorhydrate ou le bromhydrate de carboxamidine qui se produit, par une opération de séparation mécanique et le cas échéant on soumet à un retraitement thermique.

8. Procédé selon l'une quelconque des revendications précédentes,
**caractérisé en ce qu'**
on effectue la réaction d'une manière discontinue ou en continu.
